Europäisches Patentamt

European Patent Office

Office européen des brevets

(11) Numéro de publication: **0 182 682**
**A1**

## DEMANDE DE BREVET EUROPEEN

(21) Numéro de dépôt: **85402006.2**

(22) Date de dépôt: **16.10.85**

(51) Int. Cl.⁴: **A 61 M 5/32**

(30) Priorité: **18.10.84 FR 8415983**
**15.04.85 FR 8505622**

(43) Date de publication de la demande: **28.05.86**
**Bulletin 86/22**

(84) Etats contractants désignés: **DE GB IT NL**

(71) Demandeur: **Reveillon, Jean André Henri, Chemin de la Rivayrolle Montplaisir, F-81000 Albi (FR)**

(72) Inventeur: **Reveillon, Jean André Henri, Chemin de la Rivayrolle Montplaisir, F-81000 Albi (FR)**

(74) Mandataire: **Cabinet BERT, DE KERAVENANT & HERRBURGER, 115, Boulevard Haussmann, F-75008 Paris (FR)**

(54) **Dispositif à injections parentérales.**

(57) a) Dispositif à injections parentérales,

b) caractérisé en ce qu'il comporte un corps (1) avec une aiguille (2) solidaire d'un organe de verrouillage (3), montée coulissante dans le corps (1) et un tampon désinfectant (4) placé dans le corps (1) dans la trajectoire de l'aiguille (2).

c) L'invention s'applique aux dispositifs pour faire des injections successives.

EP 0 182 682 A1

1

" Dispositif à injections parentérales "

La présente invention concerne un dispositif à injections parentérales, destiné à équiper une seringue notamment pour effectuer des injections de traitements préventifs ou curatifs, en particulier sur un troupeau d'animaux.

Actuellement, les traitements préventifs ou curatifs par injections de liquide effectuées sur les animaux d'un troupeau, se font en utilisant une seringue automatique, la même aiguille servant pour plusieurs animaux, jusqu'à ce que l'extrémité de l'aiguille soit émoussée et ne permette plus d'effectuer des injections.

Or, une telle façon de procéder ne respecte pas les règles usuelles de l'asepsie ; il en résulte fréquemment l'infection de tout un troupeau à partir de quelques animaux malades.

Pour éviter de telles difficultés, la solution consisterait à aseptiser la pointe de l'aiguille entre chaque injection ou à remplacer chaque fois l'aiguille par une aiguille stérilisée. Malheureusement, aucune de ces deux solutions n'est utilisable en pratique car le vétérinaire ou le manipulateur, devrait transporter sur lui les aiguilles d'injection ou le matériel pour stériliser. Cela n'est pas envisageable puisque dans la plupart des cas, les injections se font sur place, dans les prés.

La présente invention a pour but de remédier aux inconvénients des solutions connues et se propose de créer un moyen permettant d'éviter une contamination de troupeaux d'animaux par des moyens simples, faciles à réaliser et à transporter, et n'augmentant pas la charge de travail du personnel effectuant les injections.

A cet effet, l'invention concerne un dispositif du type ci-dessus, caractérisé en ce qu'il comprend :

- un corps,

- une aiguille solidaire d'un organe de verrouillage pour être adaptée à la seringue, cette aiguille étant monté coulissante dans le corps entre une position de repos pour laquelle l'extrémité de l'aiguille est en retrait dans le corps et une position d'injection pour laquelle l'extrémité de l'aiguille est en saillie par rapport au corps,

- un tampon désinfectant placé dans le corps sur le trajectoire de l'aiguille pour être traversé par l'aiguille lorsque celle-ci est poussée en position d'injection.

Ce dispositif qui se fixe sur n'importe quelle seringue a la place d'une aiguille traditionnelle, par exemple sur des embouts de seringues de type "luer - luer lock", pour des applications vétérinaires ou humaines, tels qu'ils sont utilisés de façon quasi générale actuellement, se met en place comme une aiguille classique sans pratiquement en augmenter l'encombrement.

Lors du déplacement de l'aiguille sous l'effet de la poussée exercée sur la seringue, l'extrémité avant du corps étant appliquée contre le point où doit se faire l'injection, la pointe traverse le tampon désinfectant. L'injection se fait puis, lorsque le manipulateur relâche la pression exercée sur la seringue, un moyen de rappel tel qu'un ressort, peut repousser la seringue et, par suite, l'aiguille en position de repos. A ce moment, l'aiguille traverse une nouvelle fois le tampon désinfectant.

Suivant un mode de réalisation, la pointe de l'aiguille peut séjourner en permanence à l'intérieur du tampon désinfectant.

Ainsi, pour chaque injection, l'aiguille traverse au moins partiellement, deux fois le tampon désinfectant, une première fois lors de l'injection et une seconde fois à la fin de l'injection, ce qui évite toute contamination d'un animal à l'autre.

Suivant une autre caractéristique de l'invention, le rappel de l'aiguille en position de repos est assuré par un ressort de compression prenant appui sur le corps et sur l'aiguille. Ce ressort est avantageusement logé à l'intérieur du corps.

Suivant une autre caractéristique de l'invention, le corps est un tube muni d'un fond percé pour le passage de l'extrémité de l'aiguille et le tampon désinfectant est logé contre le fond, un piston mobile dans le corps poussant le tampon contre le fond et constituant un appui pour le ressort de rappel de l'aiguille.

Suivant un développement particulièrement intéressant, le piston est déformable élastiquement pour comprimer le tampon lors de la compression du ressort de rappel par l'aiguille venant en position d'injection.

Il existe de multiples possibilités de réalisation d'un piston déformable élastiquement et qui, sous l'effet d'une certaine compression, se déforme et comprime le tampon désinfectant de façon à faire venir le liquide sur la pointe de l'aiguille. Or, cette compression, qui est transmise par le ressort de rappel, est la plus forte lorsque l'aiguille se déplace vers la position d'injection ou revient de cette position.

De façon particulièrement avantageuse, le fond du corps est concave et la périphérie du piston est formée par une lèvre élastique située du côté du tampon et qui se déforme élastiquement dans la concavité du fond en

4

comprimant le tampon désinfectant.

La forme concave du fond du corps, combinée à la lèvre élastique déformable du piston, du côté du fond, permet au piston, sous l'effet du ressort de compression lui-même poussé par la seringue, de déformer les lèvres du piston et de diminuer ainsi le volume de la cavité dans laquelle se trouve le tampon désinfectant. Il en résulte l'expression de liquide sur la pointe de l'aiguille.

Comme la lèvre du piston est élastique, dès que l'aiguille est suffisamment relâchée, le piston revient lui-même en arrière. Il y a donc un double ressort, à savoir le ressort de rappel proprement dit et l'effet de ressort agissant dans le même sens et qui est exercé par le piston.

Cette réalisation offre, en outre, l'avantage de ne pas exercer sur le tampon désinfectant, en permanence, une pression qui ferait suinter le liquide désinfectant par le perçage du fond du corps. Cette compression n'aurait pas de conséquence grave pendant l'utilisation du dispositif, durée d'utilisation qui est relativement courte, mais pendant toute la durée de stockage du dispositif.

Suivant une autre caractéristique, le corps et l'aiguille sont munis d'un organe de butée limitant le mouvement de recul de l'aiguille par rapport au corps.

De façon particulièrement avantageuse, l'organe de butée est constitué par des collerettes à section en dents de scie prévues respectivement sur la surface intérieure du corps et sur un support solidaire de l'aiguille.

Cette réalisation est très simple, tant à la fabrication qu'au montage. En effet, l'aiguille peut être reliée à l'organe de verrouillage par l'intermédiaire d'un support en forme de bloc cylindrique correspondant sensiblement à la section du cylindre du corps, ce bloc ayant extérieurement une collerette coopérant avec une collerette prévue sur la surface intérieure du cylindre du corps.

5

Si ces collerettes ont une section en dents de scie,dont les flancs raides sont tournés l'un vers l'autre, il est facile, lors du montage, d'introduire l'aiguille et son support dans le corps en jouant sur l'effet de rampe ou effet de coin des deux collerettes.

Une fois que la collerette du support de l'aiguille a dépassé la collerette du cylindre du corps, ces deux collerettes jouent le rôle de butées, l'une venant s'appuyer contre l'autre sous l'effet du ressort de compression.

Suivant une autre caractéristique de l'invention, le tampon présente une excroissance par rapport au fond du corps.

Cette excroissance peut être solidaire du tampon désinfectant. Il peut également s'agir d'un petit tampon rapporté extérieurement sur le corps, au niveau de son orifice de façon que, par effet de capillarité et grâce à l'entraînement par l'aiguille, le liquide désinfectant imprègne cette excroissance qui, lorsqu'elle est appliquée contre la peau, laisse une trace de liquide désinfectant.

Suivant une autre caractéristique, l'ouverture du fond et/ou l'excroissance du tampon désinfectant sont recouvertes par un opercule extérieur.

Cet opercule extérieur a l'avantage, d'une part, d'éviter la contamination de l'excroissance ou du tampon désinfectant et, d'autre part, de constituer une garantie d'inviolabilité.

Suivant une autre caractéristique, l'aiguille est munie, au niveau de son organe de verrouillage, de pattes de préhension permettant de fixer l'organe de verrouillage sur l'organe correspondant de la seringue.

Malgré ces nombreux avantages du point de vue de l'asepsie, le dispositif décrit ci-dessus peut présenter l'inconvénient de ne permettre qu'une course faible de l'aiguille entre la position de repos et la position d'injection, et donc de ne pas autoriser une introduction profonde de l'aiguille dans l'organisme du sujet à traiter.

Or, on s'est aperçu que cette lacune risquait parfois de limiter, dans une large mesure, le domaine d'utilisation du dispositif.

Une variante de l'invention propose un dispositif du type ci-dessus, permettant de découvrir beaucoup plus largement l'aiguille et donc de l'introduire plus profondément dans l'organisme du sujet à traiter.

Le dispositif conforme à cette variante est caractérisé en ce que le corps est constitué par au moins trois éléments tubulaires emboîtés coulissant télescopiquement les uns dans les autres, l'un de ces éléments ou premier élément étant muni d'un fond percé pour le passage de l'extrémité de l'aiguille, contre lequel est logé le tampon désinfectant, tandis qu'un autre élément ou troisième élément, opposé au premier élément, porte l'organe de verrouillage de l'aiguille à son extrémité opposée à ce dernier.

En conséquence, conformément à cette variante, l'aiguille est solidaire du troisième élément, tandis que les premier et second éléments peuvent librement coulisser à l'intérieur de ce dernier. Cette configuration permet d'augmenter dans une large mesure l'amplitude du mouvement de l'aiguille entre la position de repos ou la position d'injection ou réciproquement, et donc, d'augmenter considérablement la profondeur d'injection.

La configuration la plus fréquente de ce dispositif consiste à prévoir un corps constitué de trois éléments tubulaires, les premier, second et troisième éléments considérés dans cet ordre ayant, respectivement, des diamètres allant en croissant ; conformément à cette configuration, en position d'injection, les premier et second éléments sont donc totalement ou partiellement repliés dans le troisième.

Selon une autre caractéristique de l'invention, le corps est muni d'un ressort de rappel monté sur ce dernier, de manière à rappeler l'aiguille en position de repos.

Il s'agit là, en fait, d'une caractéristique similaire à celle définie ci-dessus ; la configuration de l'ensemble est de préférence choisie de manière telle que, en position de repos, l'extrémité de l'aiguille se trouve à l'intérieur du tampon où elle peut être désinfectée en permanence de manière satisfaisante.

Selon une autre caractéristique de l'invention, et là encore d'une façon similaire au dispositif conforme à la première variante, le premier élément comporte un piston mobile en un matériau élastiquement déformable constituant un appui pour le ressort de rappel et comprimant le tampon contre le fond du premier élément lors de la compression de ce ressort par l'aiguille venant en position d'injection.

Il s'agit là, en fait, d'une caractéristique essentielle de l'invention qui permet, lors du déplacement de l'aiguille de la position de repos à la position d'injection, de comprimer le tampon de façon à faire venir le liquide sur la pointe de l'aiguille et de relâcher celui-ci lorsque cette dernière se déplace à nouveau vers sa position de repos sous l'effet du ressort de rappel, empêchant ainsi toute perte de liquide désinfectant.

Selon une autre caractéristique de l'invention, les trois éléments constituant le corps sont munis d'organes de butée limitant le mouvement de l'aiguille lorsque celle-ci se déplace de sa position d'injection à sa position de repos.

La présence de tels organes de butée est indispensable à la bonne marche du dispositif pour éviter toute séparation intempestive des trois éléments constituant le corps du dispositif ; de manière avantageuse, ces organes sont constitués par des collerettes à section en dents de scie, respectivement prévues sur la surface extérieure du premier élément, sur la surface intérieure et sur la surface extérieure du second élément ainsi que sur la surface intérieure du troisième élément, ces collerettes coopérant deux à deux pour limiter le déplacement de l'aiguille.

La présence de ces collerettes permet d'exclure tout risque de démontage intempestif du corps du dispositif.

La présente invention sera décrite, de façon plus détaillée, à l'aide des dessins annexés, dans lesquels :

- la figure 1 est une vue en coupe partielle d'un dispositif selon la première variante de l'invention, en position de repos,

- la figure 2 est une vue en coupe du détail II-II de la figure 1,

- la figure 3 est une vue en coupe du dispositif représenté sur la figure 1, en position d'injection,

- la figure 4 est une coupe schématique représentant le dispositif conforme à la seconde variante de l'invention, en position de repos,

- la figure 5 est une coupe schématique représentant le dispositif représenté sur la figure 4, en position d'injection.

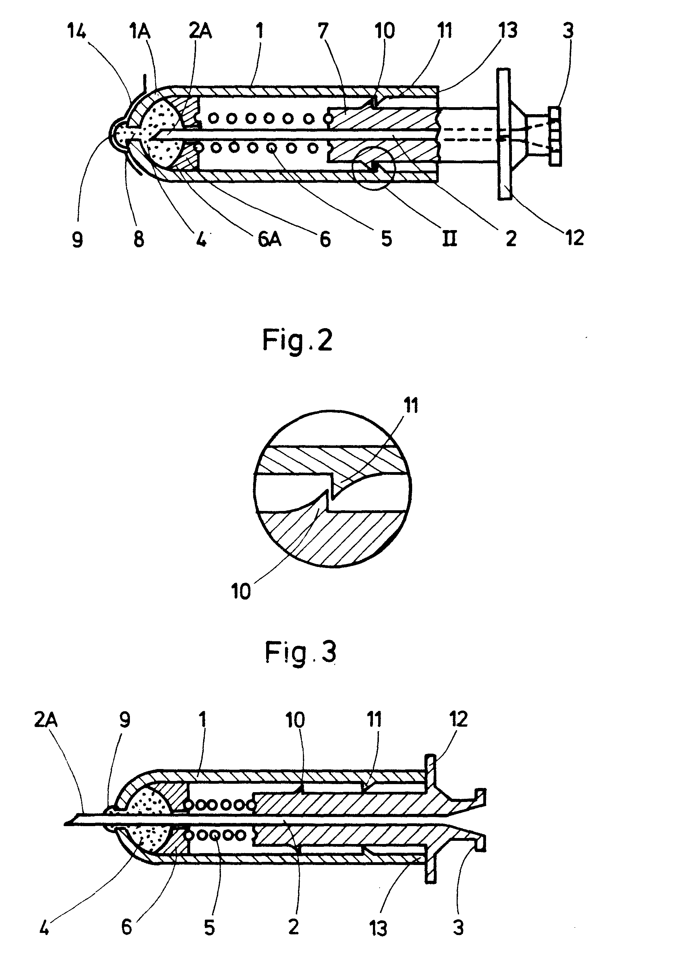

Selon la figure 1, une première variante de l'invention concerne un dispositif à injections parentérales, destiné à équiper une seringue non représentée. Ce dispositif se compose d'un corps 1 et d'une aiguille 2 solidaire d'un organe de verrouillage 3, destinée à être adaptée sur la seringue non représentée. Cette aiguille 2 est montée coulissante dans le corps 1 entre une position de repos (position représentée sur la figure 1) et une position d'injection (position représentée à la figure 3). Lorsque l'aiguille 2 est en position de repos, son extrémité 2A est en retrait par rapport au corps 1 alors qu'en position d'injection, l'extrémité 2A est en saillie par rapport au corps 1.

Ce dispositif comporte également un tampon désinfectant 4, placé sur la trajectoire de l'aiguille 2, de façon que l'aiguille 2 traverse ce tampon, au moins partiellement, lorsqu'elle passe de la position de repos (figure 1) à la position d'injection (figure 3).

Pour passer de la position de repos à la position d'injection, le manipulateur ayant appliqué l'extrémité avant 1A du corps 1 contre la peau à l'endroit choisi pour l'injection, pousse sur la seringue de façon à faire pénétrer l'extrémité 2A de l'aiguille dans la peau pour effectuer l'injection. Le mouvement inverse peut être, soit uniquement manuel, soit commandé par un ressort de rappel 5, comme représenté à la figure 1. Ce ressort de rappel 5 est un ressort de compression prenant appui sur le corps 1 par l'intermédiaire d'un piston 6 et sur l'aiguille 2 pour rappeler l'aiguille en position de repos.

En fait, l'aiguille 2 est solidaire d'un support 7, portant l'organe de verrouillage 3. Ce support 7, dont la section correspond sensiblement à la section intérieure du corps 1, sert de surface d'appui pour le ressort de compression 5.

Comme représenté aux figures, l'extrémité avant 1A du corps 1 est un fond muni d'un perçage ou orifice 8 pour le passage de l'extrémité 2A de l'aiguille.

De plus, selon le mode de réalisation représenté aux figures 1 et 3, le fond 1A du corps 1 est bombé de façon concave et le tampon désinfectant 4 est placé dans cette partie concave, par ailleurs, délimitée par le piston 6. Le ressort de compression appuie également sur le piston 6. Il est à remarquer que le piston 6 est muni d'une lèvre périphérique 6A du côté du tampon. Le piston 6 étant en un matériau déformable élastiquement sous l'effet d'une poussée exercée par la seringue sur l'aiguille 2 (et son support 7) et par le ressort 5, le piston 6 avance et la lèvre 6A se déforme élastiquement suivant la forme bombée du fond 1A.

Au cours de ce mouvement de compression, le tampon 4 est comprimé et du liquide est exprimé sur l'extrémité 2A de l'aiguille 2.

Lors du mouvement inverse, c'est-à-dire lorsque la pression exercée sur la seringue est relâchée, l'aiguille 2 revient, libère le ressort 5 qui, lui-même, libère le piston 6. Sous l'effet de l'élasticité de la lèvre 6A, le piston 6 revient dans sa position de repos, position dans laquelle le tampon désinfectant n'est pas comprimé (de façon à éviter la perte de liquide désinfectant).

Il est à remarquer, selon la figure 1, que le tampon désinfectant 4 forme une excroissance 9 devant le perçage 8, à l'extérieur du fond 1A. Cette excroissance 9 peut également être constituée par une partie de tampon, rapportée extérieurement. Cette excroissance 9 (ou partie de tampon) s'imprègne de liquide désinfectant et, lorsque le dispositif est appliqué contre la surface de la peau à l'endroit de l'injection, l'écrasement de cette excroissance 9 entraîne le dépôt de liquide désinfectant à l'endroit de l'injection, complétant ainsi l'asepsie.

Il est évident que le matériau du tampon désinfectant est choisi de façon que la pointe de l'aiguille 2 ne puisse couper des particules de ce matériau qui formeraient bouchon ou qui seraient injectés en même temps que le liquide de traitement.

Selon les figures 1 et 2, l'aiguille comporte un moyen de butée limitant sa course vers la position de repos. En fait, ce moyen de butée, constitué par une collerette 10, périphérique, prévue sur le support 7, et une collerette correspondante 11, prévue sur la surface intérieure du corps 1. Les collerettes 10, 11 ont, de préférence, une section en dents de scie dont les faces à flanc raide sont tournées l'une vers l'autre. La position respective des collerettes 10, 11 est choisie de façon à définir la position de repos. Cette position de repos est de préférence celle représentée à la figure 1, c'est-à-dire une position telle que l'extrémité 2A de l'aiguille 2 séjourne encore dans le tampon désinfectant 4.

Cette forme des collerettes 10, 11 est d'une réalisation facile et permet le montage, par simple emmanchement, en jouant sur l'élasticité des collerettes et des matériaux constituant le corps 1 et le support 7. Dès que la collerette 10 est passée dans la collerette 11, elle se déploie et ne peut plus se dégager.

La figure 2 montre, à échelle agrandie, la forme des deux collerettes 10, 11.

Selon la figure 1, le support 7 est muni de pattes de préhension 12 qui permettent de tenir et de tourner le dispositif pour permettre de tourner l'organe 3 et de le visser, par exemple par un quart de tour sur la seringue.

La position du dispositif, représentée à la figure 1, est la position de repos. Dans cette position, la pointe de l'aiguille est en retrait. La pointe est protégée. De plus, le manipulateur est, lui aussi, protégé et il ne risque pas de s'érafler ou de se piquer, voire de s'injecter quelques gouttes de liquide si, accidentellement, l'animal qu'il veut piquer, se débat.

La figure 3 montre la position d'injection du dispositif. Dans cette position d'injection, les organes de préhension 12 viennent s'appuyer contre le bord arrière 13 du corps formant butée limitant la course de l'aiguille vers l'avant.

Enfin, il convient de remarquer que le dispositif étant distribué en position de repos, c'est-à-dire l'aiguille étant rétractée, il convient de recouvrir l'excroissance 9 ou/et le perçage 9 par un opercule 14 de protection et de garantie. Cet opercule protège l'excroissance 9 et le tampon 4 contre les injections et constitue, en même temps, un témoin prouvant que le dispositif n'a pas été utilisé.

Le dispositif décrit ci-dessus se réalise avantageusement en matière plastique rigide pour le corps 1 et le support 7 de l'aiguille 2. L'aiguille 2 est réalisée en acier. Le piston 6 est, de préférence, réalisé en une matière plastique relativement souple et le tampon 4 en une mousse synthétique. Le ressort 5 est, de préférence, en acier.

Enfin, l'organe de verrouillage 3 est un organe de verrouillage, par exemple, pour un embout de seringue, de type "luer", "luer lock".

Dans un but de clarté, dans ce qui suit, les éléments du dispositif conforme à la seconde variante, similaires au éléments correspondants du dispositif selon la première variante, seront désignés par des références identiques.

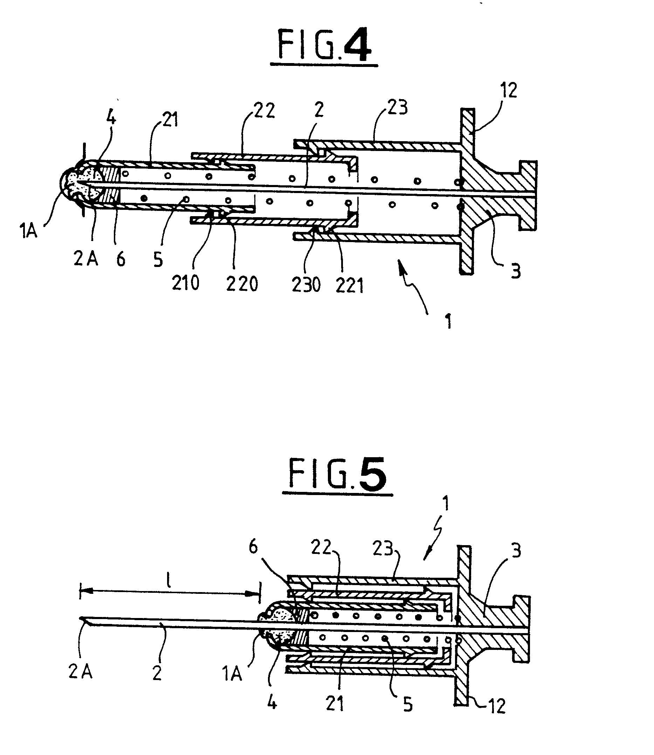

Selon les figures 4 et 5, le dispositif conforme à la seconde variante de l'invention est constitué principalement par un corps 1 ainsi que par une aiguille 2 susceptible d'être adaptée sur une seringue quelconque, non représentée, au moyen d'un organe de verrouillage 3.

L'aiguille 2 est montée coulissante dans le corps 1 entre une position de repos représentée sur la figure 4, pour laquelle son extrémité 2A est en retrait dans le corps 1 et une position d'injection, représentée sur la figure 5, pour laquelle l'extrémité 2A est en saillie par rapport au corps 1 sur une longueur $\ell$, permettant ainsi son introduction à une profondeur relativement importante dans l'organisme du sujet à traiter.

Un tampon désinfectant 4 est, par ailleurs, placé dans le corps 1 sur la trajectoire de l'aiguille 2, pour être traversée par cette dernière lorsqu'elle est poussée en position d'injection (figure 5).

Pour permettre d'avoir une amplitude de mouvements suffisante entre la position de repos (figure 4) et la position d'injection (figure 5), le corps 1 est constitué par trois éléments tubulaires 21, 22, 23 de diamètre croissant dans cet ordre, qui sont emboîtés les uns dans les autres et susceptibles de coulisser télescopiquement.

Le premier élément 21, de plus petit diamètre, est muni d'un fond 1A percé permettant le passage de l'extrémité 2A de l'aiguille 2. Le fond 1A fait, par ailleurs, office d'appui pour le tampon 4 imprégné de désinfectant, qui est maintenu par un piston mobile 6 en un matériau élastiquement déformable, susceptible de venir comprimer le tampon 4 d'une manière qui sera décrite plus en détail dans la suite de cet exposé.

Le troisième élément 23 porte l'organe de verrouillage 3 à son extrémité opposée au fond 1A du premier élément 21 et porte, par ailleurs, des organes de préhension 12 susceptibles de faciliter la manipulation du dispositif. Etant donné que celui-ci porte les organes de verrouillage 3, l'aiguille 2 est en fait solidaire du troisième élément 23.

A partir de la position d'injection représentée sur la figure 5, le dispositif est automatiquement ramené dans la position de repos représentée sur la figure 4, dès que l'on relâche toute contrainte manuelle par la force exercée par un ressort de rappel 5 qui s'appuie, d'une part, sur le piston 6 et, d'autre part, à la partie arrière du troisième élément 23.

Ce ressort 5 a, également, pour rôle de comprimer le piston 6 en position d'utilisation pour exprimer le liquide contenu dans le tampon 4 et en imprégner la pointe 2A de l'aiguille 2.

Pour que le dispositif décrit ci-dessus puisse fonctionner de manière satisfaisante, il est indispensable qu'il comporte des organes de butée limitant le mouvement des différents éléments constituant le corps 1 à la position

14

extrême représentée sur la figure 1.

A cet effet, le premier élément 21 comporte, sur sa périphérie externe, une collerette à section en dents de scie 210 coopèrant avec une collerette 220 correspondante prévue à la périphérie interne du second élément 22. Cet élément 22 comporte, par ailleurs, sur sa périphérie externe, une seconde collerette 221 à section en dents de scie qui coopère avec une collerette 230 prévue à la périphérie interne du troisième élément 23. Ces différentes collerettes coopèrent deux à deux entre-elles pour empêcher le développement du corps 1 au-delà de la position représentée sur la figure 4.

La description ci-dessus montre clairement l'avantage du dispositif objet de la seconde variante de l'invention par rapport à celui correspondant à la première variante pour ce qui est de l'amplitude possible du mouvement de l'aiguille 2.

Le dispositif selon l'invention convient par ailleurs tout particulièrement pour le traitement d'un groupe d'individus avec une seringue automatique, c'est-à-dire contenant une réserve de produits permettant d'effectuer de multiples injections sans nouveau remplissage. Bien que la description ci-dessus mentionne l'application la plus fréquente, à savoir l'usage dans le domaine vétérinaire, cela n'exclut pas l'application à l'homme.

R E V E N D I C A T I O N S

1°) Dispositif à injections parentérales, destiné à être fixé sur une seringue quelconque à la place d'une aiguille traditionnelle, dispositif caractérisé en ce qu'il comprend :

- un corps tubulaire (1) muni d'un fond percé (1A),

- une aiguille (2) solidaire d'un organe de verrouillage (3) pour être adaptée à la seringue, cette aiguille (2) étant montée coulissante dans le corps (1) entre une position de repos pour laquelle l'extrémité (2A) de l'aiguille est en retrait dans le corps (1) et une position d'injection pour laquelle l'extrémité (2A) de l'aiguille est en saillie par rapport au fond (1A) du corps (1),

- un ressort de compression (5) prenant appui sur le corps (1) et sur l'aiguille (2) pour rappeler l'aiguille (2) en position de repos,

- un tampon désinfectant (4) logé dans le corps (1) contre le fond (1A) sur la trajectoire de l'aiguille (2) pour être traversé par l'aiguille (2) lorsque celle-ci est poussée en position d'injection,

- un piston (6) mobile dans le corps (1) constituant un appui pour le ressort (5) et comprimant le tampon (4) contre le fond (1A) du corps (1) lors de la compression du ressort (5) par l'aiguille (2) venant en position d'injection.

2°) Dispositif selon la revendication 1, caractérisé en ce que le piston (6) est déformable élastiquement.

3°) Dispositif selon la revendication 2, caractérisé en ce que le fond (1A) du corps (1) est concave et la périphérie du piston (6) est formée par une lèvre élastique (6A) située du côté du tampon (4) et qui se déforme élastiquement dans la concavité du fond (1A) en comprimant le tampon désinfectant (4).

4°) Dispositif selon la revendication 1, caractérisé en ce que le corps (1) et l'aiguille (2) sont munis d'un organe de butée (10, 11) limitant le mouvement de recul de l'aiguille (2) par rapport au corps (1).

5°) Dispositif selon la revendication 4, caractérisé en ce que l'organe de butée est constitué par des collerettes (10, 11) à section en dents de scie prévues, respectivement, sur la surface intérieure du corps (1) et sur un support (7) solidaire de l'aiguille (2).

6°) Dispositif selon l'une quelconque des revendications 1 à 5, caractérisé en ce que le tampon (4) présente une excroissance (9) par rapport au fond (1A) du corps (1).

7°) Dispositif selon l'une quelconque des revendications 1 à 6, caractérisé en ce que l'ouverture (8) du fond et/ou l'excroissance (9) du tampon désinfectant (4) sont recouverts par un opercule extérieur (14).

8°) Dispositif selon la revendication 1, caractérisé en ce que l'aiguille (2) est munie au niveau de son organe de verrouillage (3) de pattes (12) de préhension permettant de fixer l'organe de verrouillage (3) sur l'organe correspondant de la seringue.

9°) Dispositif selon l'une quelconque des revendications 1 à 8, caractérisé en ce que le corps (1) est constitué par au moins trois éléments tubulaires (21, 22, 23) emboîtés coulissant télescopiquement les uns dans les autres, l'un de ces éléments (21) ou premier élément étant muni d'un fond (1A) percé pour le passage de l'extrémité (2A) de l'aiguille (2) contre lequel est logé le tampon désinfectant (4) tandis qu'un autre élément ou troisième élément (23) opposé au premier élément (21) porte l'organe de verrouillage (3) de l'aiguille (2) à son extrémité opposée à ce dernier.

10°) Dispositif selon la revendication 9, caractérisé en ce que les permier, second et troisième éléments (21, 22, 23) du corps (1) considérés dans cet ordre, ont respectivement des diamètres allant en croissant.

11°) Dispositif selon l'une quelconque des revendications 9 et 10, caractérisé en ce que le premier élément (21) comporte le piston mobile (6) en un matériau élastiquement déformable qui constitue un appui pour le ressort de rappel (5) et comprime le tampon (4) contre le fond (1A) du premier élément (21) lors de la compression de ce ressort (5) par l'aiguille (2) venant en position d'injection.

12°) Dispositif selon l'une quelconque des revendications 9 à 11, caractérisé en ce que les trois éléments (21, 22, 23) constituant le corps (1), sont munis d'organes de butée (210, 220, 221, 230) limitant le mouvement de l'aiguille (2) lorsque celle-ci se déplace de sa position d'injection à sa position de repos.

13°) Dispositif selon la revendication 12, caractérisé en ce que les organes de butée (210, 220, 221, 230) sont constitués par des collerettes à section en dents de scie respectivement prévues sur la surface extérieure du premier élément (21), sur la surface intérieure et sur la surface extérieure du second élément (22) ainsi que sur la surface intérieure du troisième élément (23), ces collerettes (210, 220 et 221, 230) coopèrant deux à deux pour limiter le déplacement de l'aiguille (2).

0182682

## Fig.1

## Fig.2

## Fig.3

C182682

## FIG.4

## FIG.5

Office européen
des brevets

**RAPPORT DE RECHERCHE EUROPEENNE**

C1828

Numéro de la demande

EP 85 40 2006

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-2 876 770 (R. WHITE)<br><br>* Colonne 3, lignes 33-73; figures 3,4 * | 1-6,11 ,12 | A 61 M 5/32 |
| Y | US-A-4 240 429 (SHACKELFORD)<br><br>* Colonne 4, ligne 44 - colonne 5, ligne 2; figures 2,3 * | 1-6,11 ,12 | |
| A | GB-A-2 080 689 (H. DENT)<br>* Figure 1 * | 1 | |
| A | GB-A-2 079 607 (TEMPLE COX DEVELOPMEMT)<br>* Page 1, lignes 116,117; figure 1 * | 7 | |
| A | US-A-3 368 559 (SARNOFF & STANLEY)<br>* Figure 4 * | 5,13, 12 | DOMAINES TECHNIQUES RECHERCHES (Int. Cl.4)<br><br>A 61 M |
| A | US-A-2 664 086 (G. TRANSUE)<br>* Figures 1,2 * | 9,10 | |
| A | FR-A-2 210 419 (SGE RESEARCH PROPRIETARY LTD.)<br>* Revendication 1; figures 1,2 * | 9 | |

Le présent rapport de recherche a été établi pour toutes les revendications

| Lieu de la recherche | Date d'achèvement de la recherche | Examinateur |
|---|---|---|
| LA HAYE | 20-01-1986 | EHRSAM F.J.A. |